Europäisches Patentamt

European Patent Office

Office européen des brevets

(11) Publication number: **0 356 739 B1**

# EUROPEAN PATENT SPECIFICATION

(45) Date of publication of patent specification: **13.12.95**

(21) Application number: **89114207.7**

(22) Date of filing: **01.08.89**

(51) Int. Cl.⁶: **C12N 15/52**, C12N 15/77,
C12N 1/20, C12P 13/06,
C12P 13/08, //(C12N15/52,
C12R1:13,1:15)

(54) Recombinant DNA, microorganism carrying said recombinant DNA, and process for producing L-amino acids by the use of said microorganism

(30) Priority: **03.08.88 JP 194031/88**

(43) Date of publication of application:
**07.03.90 Bulletin 90/10**

(45) Publication of the grant of the patent:
**13.12.95 Bulletin 95/50**

(84) Designated Contracting States:
**DE FR NL**

(56) References cited:
**EP-A- 0 179 338
EP-A- 0 183 175
EP-A- 0 190 921
EP-A- 0 233 581
WO-A-87/02984**

(73) Proprietor: **AJINOMOTO CO., INC.**
**5-8, Kyobashi 1-chome, Chuo-ku**
**Tokyo 104 (JP)**

(72) Inventor: **Sato, Katsuaki**
**No. 1-1, Suzuki-cho**
**Kawasaki-ku**
**Kawasaki-shi**
**Kanagawa-ken (JP)**
Inventor: **Yoshino, Eriko**
**No. 1-1, Suzuki-cho**
**Kawasaki-ku**
**Kawasaki-shi**
**Kanagawa-ken (JP)**
Inventor: **Hashiguchi, Kenichi**
**No. 1-1, Suzuki-cho**
**Kawasaki-ku**
**Kawasaki-shi**
**Kanagawa-ken (JP)**

AGRICULTURAL AND BIOLOGICAL CHEMIS-
TRY, vol. 39, no. 11, November 1975,
pages2193-2198, Tokyo, JP; T. TSUCHIDA et
al.: "Genetic changes of re-
gulatorymechanisms occurred in leucine
and valine producing mutants derived from
Brevibacterium lactofermentum 2256"

DEVELOPMENTS IN INDUSTRIAL MICROBI-
OLOGY, vol. 24, 1983, pages 109-119,Wash-
ington, D.C., US; H. MOMOSE: "New genetic
approaches to amino-acid-producing
strains"

BIO/TECHNOLOGY, vol. 5, no. 2, February
1987, pages 137-145, New York, US;
J.F.MARTIN et al.: "Cloning systems in amino
acid-producing corynebacteria"

Inventor: **Enei, Hitoshi**
**No. 1-1, Suzuki-cho**
**Kawasaki-ku**
**Kawasaki-shi**
**Kanagawa-ken (JP)**

(74) Representative: **Strehl Schübel-Hopf Groening**
**& Partner**
**Maximilianstrasse 54**
**D-80538 München (DE)**

**Description**

This invention relates to recombinant DNA having the gene for acetohydroxy acid synthase derived from the genus Brevibacterium incorporated therein, and to a process for producing L-valine, L-leucine or L-isoleucine by the use of a microorganism capable of producing one of these amino acids that carries said recombinant DNA.

[ Prior Art ]

For the manufacture of L-valine, L-leucine and L-isoleucine by the fermentation process, has been adopted the method in which a wild strain is imparted with the ability of producing one of these amino acids by artificial mutation, because wild strains scarcely produce these amino acids outside the microbial cells.

As the artificial mutants capable of producing L-valine, L-leucine or L-isoleucine, are known those belonging to the genus Brevibacterium, Corynebacterium or Serratia and resistant to antagonist against these amino acids. To be more specific, the method consists in the use of mutants in which acetohydroxy acid synthase ( the key enzyme participating in the production of L-valine, L-leucine or L-isoleucine ) is less subject to feedback inhibition by its end product ( L-valine, L-leucine and/or L-isoleucine ) or antagonist thereof.

Such mutants as mentioned above have the problem that only one gene that codes for acetohydroxy acid synthase is involved in each genome, and hence its enzymatic activity is limited even when released from the feedback inhibition. In addition, it is not an easy task to release each strain from the feedback inhibition.

EP-A-0 183 175 relates to a process for producing an aromatic amino acid which comprises culturing a Coryneform bacterium carrying a recombinant DNA constructed by connecting a gene coding for 3-deoxy-D-arabino-heptulonic acid-7-phosphate-synthetase with a plasmid vector capable of propagating in a Coryneform bacterial cell.

Agr. Biol. Chem. 39 (11) (1975), 2193-2198 shows genetic changes of regulatory mechanisms occurring in leucine and valine producing mutants of a Brevibacterium lactofermentum strain. Regulatory mechanisms are compared between 2-thiazolealanine resistant L-leucine and L-valine producing mutants and the 2-thiazolealanine sensitive original strains of Brevibacterium lactofermentum.

WO 87/02984 relates to a method for genetically engineering microbial strains which produce amino acids and other specialty chemicals. The method comprises molecular cloning of DNA fragments containing the total genes involved in the biosynthesis of said chemicals while removing biochemical and genetic regulation controlling mechanisms. The biogenetic cassette is cloned using a vector molecule and transformed into appropriate recipient strains.

[ Problems to be Solved by the Invention ]

The object of this invention is to provide a method of artificially controlling or nullifying the metabolic regulation in the L-valine, L-leucine or L-isoleucine biosynthesis system, thereby eliminating the defect of limited number of genes associated with the conventional method, and facilitating the release from feedback inhibition. To put it concretely, the method proposed in this invention is intended to release acetohydroxy acid synthase ( the key enzyme common to the biosynthesis of L-valine, L-leucine and L-isoleucine ) from feedback inhibition by these amino acids and to increase the number of genes for this enzyme in each genome.

[ Means to Solve the Problems ]

Intensive studies on the above-mentioned problems have led us to find that the L-valine, L-leucine or L-isoleucine productivity can be enhanced by isolating from a microorganism belonging to the genus Brevibacterium, a DNA fragment that codes for acetohydroxy acid synthase, incorporating this fragment into a plasmid capable of autonomic multiplication, and introducing this recombinant plasmid into a coryneform glutamate-producing bacterium ( e.g., Brevibacterium lactofermentum ).

The DNA fragment carrying the gene for acetohydroxy acid synthase can be obtained from the chromosomal DNA of a microorganism of Brevibacterium. Such microorganisms from which a DNA carrying genetic information ( e.g., DNA fragment carrying the gene for acetohydroxy acid synthase ) can be obtained are hereinafter referred to as DNA donors.

3

As the DNA donor, microorganisms belonging to the genus Brevibacterium can be used which show a relatively high activity in the production of L-valine, L-leucine or L-isoleucine. Such microorganisms are known to a person skilled in the art. Preferably, there may be used those mutants which have been imparted with resistance to antagonists of L-valine, L-leucine or L-isoleucine and hence show higher biosynthetic activity for these amino acids or precursors thereof. Preferred examples of such DNA donors are microorganisms of the species Brevibacterium lactofermentum which have high biosynthetic activity for L-valine, L-leucine or L-isoleucine or precursors thereof. A specific example is a L-valine producing strain of Brevibacterium lactofermentum. Mutants having high acetohydroxy acid synthase activity which show no feedback inhibition by the end product (L-Val, L-Leu and L-Ile) can particularly be obtained by using the method described in Proceedings of the First International Congress of IAMS, 363-372p, 1975, Haruo Momose, et al. As examples of the antagonists of these amino acids, may be mentioned $\alpha$-amino-$\beta$-hydroxyvaleric acid, aminohexylisovaleric acid, isoleucine hydroxamate, 2-thiazole-alanine, $\beta$-hydroxyleucine, trichloroalanine and $\alpha$-methyllysine.

The gene for acetohydroxy acid synthase may be obtained by a process comprising, as a first step, isolating the chromosomal DNA from a DNA donor. The isolation of the chromosomal DNA can, for example, be performed by culturing a suitable DNA donor microorganism, for example a microorganism of the species Brevibacterium lactofermentum having high biosynthetic activity for L-valine, L-leucine or L-isoleucine or precursors thereof in a suitable culture medium. In a preferred embodiment, the DNA donor microorganism, for example a L-valine-producing strain of Brevibacterium lactofermentum, is inoculated in a suitable culture medium containing N-sources, C-sources and minerals, for example polypeptone, glucose and sodium chloride, over an extended period of time at normal or slightly elevated temperatures, whereafter the grown cells are collected at the stage of multiple propagation. The obtained microbial cells are lysed in a per se known manner, whereafter the chromosomal DNA is extracted and purified by methods known per se.

The obtained chromosomal DNA thereafter is properly cleaved. This can be performed in the manner also known per se by suitable restriction enzymes which are selected in accordance with the restriction site. As a specific preferred example of the present application, the chromosomal DNA obtained from a Brevibacterium lactofermentum culture is partially cleaved at the Sau3 A I site. Fragments of 2 to 6 Kb were cut out from the chromosomal DNA.

The further step for obtaining the gene for acetohydroxy acid synthase consists of inserting the DNA fragments as obtained in the previously described steps into a suitable plasmid. Plasmids are considered to be "suitable" plasmids if they have the capability of autonomic multiplication in coryneform bacterial cells, for example cells of the genus Brevibacterium.

The subsequent step of the overall process consists of introducing the resulting recombinant plasmid into a microorganism of Brevibacterium defective in acetohydroxy acid synthase, and picking out a transformant imparted with the ability of producing acetohydroxy acid synthase.

The gene for acetohydroxy acid synthase is then inserted into a vector plasmid capable of automatic multiplication in coryneform bacterial cells, e.g. cells of Brevibacterium. The recombinant plasmid thus obtained is introduced into a receptor microorganism strain, e.g. into cells of Brevibacterium, thereby forming a transformant with improved productivity of L-valine, L-leucine or L-isoleucine.

As the gene for acetohydroxy acid synthase, may be used either a wild-type gene or a mutant-type gene. As the latter type, the most preferred is the one so mutated as to code for acetohydroxy acid synthase with reduced feedback inhibition by L-valin, L-leucine or L-isoleucine.

A mutant-type gene may be obtained by directly subjecting a DNA donor to mutation treatment. However, it can be obtained at a higher probability by inserting the gene for acetohydroxy acid synthase into a vector plasmid, introducing the recombinant DNA thus formed into host cells, and mutating the transformant thus obtained. Furthermore, the mutant-type gene can be obtained at the highest probability if the above recombinant DNA is subjected to in-vitro mutation treatment.

The mutant-type gene that codes for acetohydroxy acid synthase with reduced feedback inhibition by L-valine, L-leucine or L-isoleucine can be sorted out by selecting a DNA donor strain or a transformant imparted with resistance to antagonist of these amino acids ( after obtaining transformants when the recombinant DNA is subjected to mutation treatment and the mutated recombinant DNA thus formed is introduced into host cells ).

In order to confirm that the DNA donor strain or transformant imparted with resistance to antagonist contains a mutant-type gene that codes for acetohydroxy acid synthase with reduced feedback inhibition by L-valine, L-leucine or L-isoleucine, it is necessary to prepare an enzyme solution from that antagonist-resistant strain or transformant, to allow this solution to act upon pyruvic acid ( as substrate ) in the presence of L-valine, L-leucine or L-isoleucine, and to measure the amount of 2-acetolactic acid formed. An enzyme solution that shows high activity of forming 2-acetolactic acid even at a high concentration of L-valine, L-leucine or L-isoleucine is regarded as containing acetohydroxy acid synthase with reduced feedback inhibition by these amino acids.

Mutation of the DNA donor, transformant or recombinant DNA may be effected by treatment with a mutagen, such as N-methyl-N'-nitro-N-nitrosoguanidine and sulfurous acid, or by irradiation with X-rays, UV-rays or $\gamma$-rays. These methods are also efficient for mutating any genes other than that for acetohydroxy acid synthase.

As the promotor operator for acetohydroxy acid synthase gene, may be used either a wild-type operator or a mutant-type operator. It is also possible to use any powerful promotors which are known to efficiently express other types of genes. The promotor operator DNA may be the one derived from other types of living bodies or the one chemically synthesized. Furthermore, the mutant-type acetohydroxy acid synthase formed in plasmid may be introduced again into the chromosome of host cells, and the same applies to the gene derived from other types of living bodies or chemically synthesized.

[ Eamples ]

The following examples will further illustrate the invention.

Example 1

(1) Preparation of chromosomal DNA

L-valine-producing strain of Brevibacterium lactofermentum was inoculated to 50 ml of CM2G medium ( containing 10 g polypeptone, 10 g Y.E., 5 g glucose and 5 g NaCl ), shake culture was continued overnight at 31ºC, and the grown cells were collected at the stage of multiple propagation. One gram of the microbial cells thus obtained were lysed with lysozyme•SDS, and the chromosomal DNA was extracted and purified by the phenol method, giving 5 mg of final product.

(2) Preparation vector DNA

pAJ220 ( 5445 bp ) described in JP 012284/88 was used as vector, and its DNA was prepared by the usual method.

(3) Insertion of chromosomal DNA into vector

The chromosomal DNA obtained in (1) above ( 30 $\mu$g ) was partially cleaved at Sau3A I site, and fragments of 2 to 6 kb were further cut out. Separately, 1 $\mu$g of the vector DNA was completely cleaved with restriction endonuclease BamH I. The two types of fragments thus obtained were linked together by using Takara ligation kit.

(4) Cloning of acetohydroxy acid synthase gene

Brevibacterium lactofermentum AJ12403 (FERM P-10140, FERM BP-2507) defective in acetohydroxy acid synthase activity (a strain requiring both L-isoleucine and L-valine) was used as the receptor strain.

Transformation was carried out according to the protoplast method described in Japanese published unexamined patent no. 149082/1986, and four strains imparted with resistance to trimethoprim and rendered isoleucine- and valine- independent were obtained from the transformants.

(5) Analysis of plasmids in the transformants

Each of the four strains obtained above was lysed with lysozyme•SDS and DNA was recovered from the resulting solution.

Analysis of plasmid DNA by electrophoresis on agarose revealed that each is a plasmid larger than pAJ220 and having a DNA fragment of about 3 kb incorporated therein. One of these plasmids was named pAJ220V3.

(6) Retransformation

Brevibacterium lactofermentum AJ12403 was again transformed by plasmid pAJ220V3. All of the transformants picked out on the basis of trimethoprim resistance were found to be independent on L-isoleucine and L-valine, indicating that the above-mentioned DNA fragment of about 3 kb carries the gene for acetohydroxy acid synthase.

(7) Acetohydroxy acid synthase activity of transformants

A strain being tested was cultivated in production medium (A) for about 18 hours, the grown cells were ultrasonicated, and the resulting solution was centrifuged at 15,000 r.p.m. for 20 minutes.

The supernatant thus obtained was used as a crude enzyme solution, which was added to a solution containing 65mM phosphate buffer and 100mM sodium pyruvate to effect enzymic reaction. Acetolactic acid thus formed was converted to acetoin, and the color developed by addition of 2-naphthol was measured at 530 nm. The result obtained is shown in Table 1.

It was demonstrated that cloned gene for acetohydroxy acid synthase has been incorporated in pAJ220V3.

Table 1

| Strain | Amino acid added (5mM) | | | |
|---|---|---|---|---|
| | None | L-Ile | L-Leu | L-Val |
| AJ12403 FERM BP-2507 | N.D. | — | — | — |
| AJ12403/pAJ220V3 | 100 | 101 | 119 | 108 |
| ATCC13869 | 100 | 60 | 67 | 60 |
| N.D.: Not deteced. The above figures represent relative activity values (%) when the activity in the absence of amino acid is taken as 100. | | | | |

(8) Restriction enzyme cleavage map for plasmid pAJ220V3

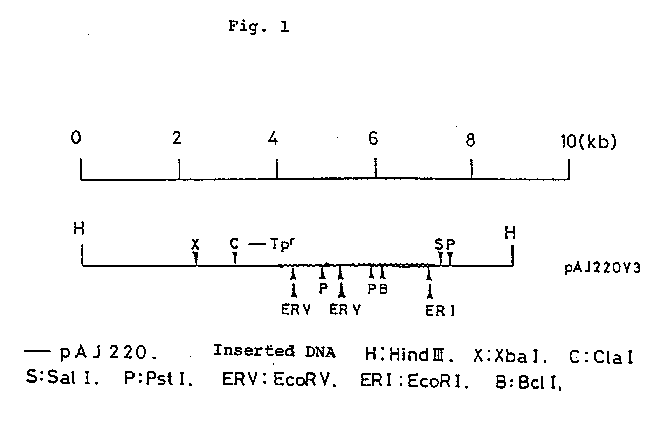

The inserted DNA fragment of about 3 kb involves at least two sites for EcoRv and Pst I each, and one site for Bcl I, EcoR I and Bal I each. A simple restriction enzyme cleavage map illustrating these sites is shown in Figure 1.

Example 2

(1) L-valine productivity of transformant derived from wild-type strain

AJ12404 (FERM P-10141, FERM BP-2508) and AJ12405 (FERM P-10142, FERM BP-2516), obtained by transforming ATCC13869 ( a wild-type strain of Brevibacterium lactofermentum ) and AJ1510 ( a wild-type strain of Brevibacterium flavum ) respectively with pAJ200V3, showed outstanding L-valine productivity as can be seen from Table 2.

6

Table 2

| Strain | L-valine (g/l)* |
|---|---|
| ATCC13869/pAJ220V3  (AJ12404 FERM BP-2508) | 7 |
| AJ1510/pAJ220V3  (AJ12405 FERM BP-2516) | 11 |
| ATCC13869 | 0 |
| AJ1510 | 0 |

* Production medium (A): Glucose ( 100 g ), ammonium sulfate ( 45 g ), monopotassium phosphate ( 1 g ), magnesium sulfate ( 1 g ), $FeSO_4 \cdot 7H_2O$ ( 0.01 g ), $MnSO_4 \cdot 7H_2O$

( 0.01 g ), Mamenou (extracted of soybean hydrolysed with HCl) ( 0.15 g; total nitrogen), biotin (100 µg ) and vitamin B1 hydrochloride (300 µg ) were dissolved in distilled water, the pH was adjusted to 7.2 by addition of KOH, water was further added to make up a total volume of one liter, and the resulting mixture was dispensed in 50-ml Sakaguchi flasks ( 20 ml in each ) and subjected to autoclave sterilization ( 115°C x 10 minutes ), followed by addition of dry-sterilized calcium carbonate ( 1 g each ).

Example 3

(1) L-isoleucine productivity in the presence of threonine deaminase

Into a wild-type strain of Brevibacterium flavum AJ1510 , were introduced pAJ220V3 and pDR1A4 ( a plasmid carrying threonine deaminase gene ( ilvA* ) of Escherichia coli desensitized against L-isoleucine ), giving a strain AJ12406 (FERM P-10143, FERM BP-2509) containing these two types of plasmids.

As can be seen from Table 3, AJ12406 showed increased relative activity for both threonine demainase ( TD ) and acetohydroxy acid synthase ( AHAS ) compared with the wild-type strain. It also showed higher L-isoleucine productivity, as shown in Table 4, compared with the transformants carrying only one of the above plasmids.

Table 3

| Strain | TD (nmol/min/mg) | AHAS (nmol/min/mg) |
|---|---|---|
| AJ1510/pDR1A4,pAJ200V3 ( AJ12406, FERM BP-2509) | 157.2 | 3.65 |
| AJ1510/pAJ220V3 ( AJ12405 FERM BP-2516) | 12.8 | 5.11 |
| AJ1510/pDR1A4 ( AJ12358 ) | 135.7 | 1.04 |
| AJ1510 | 35.2 | 1.10 |

Table 4

| Strain | L-isoleucine (g/l)* |
|---|---|
| AJ1510/pDR1A4,pAJ220V3 ( AJ12406, FERM BP-2509) | 4.6 |
| AJ1510/pAJ220V3 ( AJ12405, FERM BP-2516) | 2.4 |
| AJ1510/pDR1A4 ( AJ12358 ) | 2.5 |
| AJ1510 | 0 |

* Production medium (A): Glucose ( 100 g ), ammonium sulfate ( 50 g ), monopotassium phosphate ( 1 g ), magnesium sulfate ( 0.4 g ), $FeSO_4 \cdot 7H_2O$ ( 0.01 g ), $MnSO_4 \cdot 7H_2O$ ( 0.01 g), Mamenou (extracted of soybean hydrolysed with HCl ( 0.6 g; total nitrogen), biotin ( 50 µg ) and vitamin $B_1$ hydrochloride ( 3 mg ) were dissolved in distilled water, the pH was adjusted to 8.3 by addition of KOH, water was further added to make up a total volume of one liter, and the resulting mixture was dispensed in 50-ml Sakaguchi flasks ( 20 ml in each) and subjected to autoclave sterilization ( 115°C x 15 minutes ), followed by addition of dry-sterilized calcium carbonate ( 1 g each ).

Brevibacterium flavum AJ 12405, Brevibacterium flavum AJ 12406, Brevibacterium lactofermentum AJ 12403 and Brevibacterium lactofermentum AJ 12404 were originally deposited on July 20, 1988 at the Fermentation Research Institute, Agency of Industrial Sciences and Technology, Ministry of International Trade Industry (FRI), 1-3, Higashi 1-Chome, Tukuba-Shi, Ibaragi-Ken 305, Japan, and were accorded FERM P-10142, FERM P-10143, FERM P-10140 and FERM P-10141, respectively.

The microorganisms FERM P-10143, FERM P-10140 and FERM P-10141 were then converted into deposits under the Budapest Treaty on July 10, 1989, and were accorded FERM BP-2509, FERM BP-2507 and FERM BP-2508, respectively. The microorganism FERM P-10142 was also converted into deposit under Budapest Treaty on July 12, 1989, and was accorded FERM BP-2516.

EP 0 356 739 B1

**Claims**

1. A plasmid or phage capable of autonomic multiplication having the gene for acetohydroxy acid synthetase derived from a microorganism belonging to the genus Brevibacterium, incorporated therein.

2. A plasmid or phage as defined in claim 1 wherein said gene for acetohydroxy acid synthetase is a wild-type gene.

3. A plasmid or phage as defined in claim 1 wherein said gene for acetohydroxy acid synthetase is a mutated gene released from feedback inhibition.

4. A plasmid or phage as defined in claim 1 wherein said gene for acetohydroxy acid synthetase is under the control of its inherent promotor or of a promotor for another type of gene.

5. A plasmid or phage as defined in claim 1 wherein said gene for acetohydroxy acid synthetase is under the control of its inherent promotor or of a promotor for another type of gene resistant to trimethoprim.

6. A microorganism carrying a plasmid or phage as defined in any of the claims 1 to 5.

7. A microorganism as defined in claim 6, said microorganism being a coryneform, glutamate-producing bacterium.

8. A microorganism as defined in any of the claims 6 or 7 which microorganism belongs to the genus Brevibacterium.

9. A microorganism as defined in claim 8 which microorganism belongs to a species selected from Brevibacterium flavum and Brevibacterium lactofermentum.

10. A microorganism as defined in any of the claims 6 to 9 which microorganism is capable of producing valine, leucine or isoleucine.

11. A microorganism as defined in any of the claims 6 to 10 which microorganism is free from repression of valine, leucine or isoleucine biosynthesis.

12. A microorganism as defined in any of the claims 6 to 11 said microorganism being Brevibacterium lactofermentum.

13. A microorganism as defined in claim 12 said microorganism is Brevibacterium lactofermentum FERM-P 10141 (FERM-BP 2508).

14. A microorganism as defined in any of the claims 6 to 11 said microorganism being Brevibacterium flavum.

15. A microorganism as defined in claim 14 said microorganism is Brevibacterium flavum FERM-P 10142 (FERM-BP 2516) or Brevibacterium flavum FERM-P 10143 (FERM-BP 2509).

16. A microorganism as defined in any of the claims 7, 10 or 11, said microorganism being Corynebacterium glutamicum.

17. A microorganism with the gene for acetohydroxy acid synthetase, said gene having been transferred from the plasmid according to claim 1 to the chromosome.

18. A microorganism as defined in claim 17 said microorganism being Brevibacterium lactofermentum.

19. A microorganism as defined in claim 17 said microorganism being Brevibacterium flavum.

20. A microorganism as defined in claim 17 said microorganism being Coynebacterum glutamicum.

9

**21.** A microorganism as defined in any of the claims 6 to 20 wherein an additional plasmid or phage is contained which has the effect of increasing the productivity of valine, leucine or isoleucine.

**22.** A microorganism as defined in claim 21 wherein the additional plasmid can coexist with the plasmid as defined in claim 1.

**23.** A recombinant DNA comprising the gene coding for acetohydroxy acid synthetase, wherein said gene coding for acetohydroxy acid synthetase is obtainable from a microorganism belonging to the genus Brevibacterium having high biosynthetic activity for valine, leucine or isoleucine or precursors thereof.

**24.** A process for producing L-valine, L-leucine or L-isoleucine which comprises growing a microorganism as defined in any of the claims 6 to 22 and recovering the objective amino acid from the culture liquor.

**25.** A process for producing L-valine or L-isoleucine according to claim 24, wherein said microorganism is Brevibacterium flavum FERM P-10142 (FERM-BP 2516)), Brevibacterium flavum FERM P-10143 (FERM-BP 2509) or Brevibacterium lactofermentum FERM P-10141 (FERM-BP 2508).


**Patentansprüche**

**1.** Zur autonomen Vermehrung befähigtes Plasmid oder befähigter Phage, in das bzw. in den das Gen für Acetohydroxysäure-Synthetase eingefügt ist, das von einem Mikroorganismus des Genus Brevibacterium abgeleitet ist.

**2.** Plasmid oder Phage gemäß Anspruch 1, wobei das Gen für Acetohydroxysäure-Synthetase das Gen eines Wild-Typs ist.

**3.** Plasmid oder Phage gemäß Anspruch 1, wobei das Gen für Acetohydroxysäure-Synthetase ein von der Rückkopplungshemmung befreites, mutiertes Gen ist.

**4.** Plasmid oder Phage gemäß Anspruch 1, wobei das Gen für Acetohydroxysäure-Synthetase unter der Kontrolle seines eigenen Promotors oder eines Promotors für einen anderen Gentyp steht.

**5.** Plasmid oder Phage gemäß Anspruch 1, wobei das Gen für Acetohydroxysäure-Synthetase unter der Kontrolle seines eigenen Promotors oder eines Promotors für einen anderen Gentyp mit Resistenz gegen Trimethoprim steht.

**6.** Mikroorganismus, der ein Plasmid oder einen Phagen gemäß der Definition in einem der Ansprüche 1 bis 5 enthält.

**7.** Mikroorganismus gemäß Anspruch 6, der ein coryneformes Glutamat produzierendes Bacterium ist.

**8.** Mikroorganismus gemäß einem der Ansprüche 6 oder 7, der dem Genus Brevibacterium angehört.

**9.** Mikroorganismus gemäß Anspruch 8, der der Spezies Brevibacterium flavum oder Brevibacterium lactofermentum angehört.

**10.** Mikroorganismus gemäß einem der Ansprüche 6 bis 9, der zur Bildung von Valin, Leucin oder Isoleucin befähigt ist.

**11.** Mikroorganismus gemäß einem der Ansprüche 6 bis 10, der frei von der Repression der Valin-, Leucin- oder Isoleucin-Biosynthese ist.

**12.** Mikroorganismus gemäß einem der Ansprüche 6 bis 11, der Brevibacterium lactofermentum ist.

**13.** Mikroorganismus gemäß Anspruch 12, der Brevibacterium lactofermentum FERM-P 10141 (FERM-BP 2508) ist.

**14.** Mikroorganismus gemäß einem der Ansprüche 6 bis 11, der Brevibacterium flavum ist.

**15.** Mikroorganismus gemäß Anspruch 14, der Brevibacterium flavum FERM-P 10142 (FERM-BP 2516) oder Brevibacterium flavum FERM-P 10 143 (FERM-BP 2509) ist.

**16.** Mikroorganismus gemäß einem der Ansprüche 7, 10 oder 11, der Corynebacterium glutamicum ist.

**17.** Mikroorganismus mit dem Gen für Acetohydroxysäure-Synthetase, wobei das Gen von dem Plasmid nach Anspruch 1 in das Chromosom transferiert worden ist.

**18.** Mikroorganismus nach Anspruch 17, der Brevibacterium lactofermentum ist.

**19.** Mikroorganismus nach Anspruch 17, der Brevibacterium flavum ist.

**20.** Mikroorganismus nach Anspruch 17, der Corynebacterium glutamicum ist.

**21.** Mikroorganismus, gemäß einem der Ansprüche 6 bis 20, in dem ein zusätzliches Plasmid oder ein zusätzlicher Phage mit der Wirkung einer Erhöhung der Produktivität von Valin, Leucin oder Isoleucin vorliegt.

**22.** Mikroorganismus nach Anspruch 21, wobei das zusätzliche Plasmid mit dem in Anspruch 1 definierten Plasmid coexistieren kann.

**23.** Rekombinante DNS, die das für Acetohydroxysäure-Synthetase kodierende Gen enthält, wobei das für Acetohydroxysäure-Synthetase kodierende Gen aus einem Mikroorganismus des Genus Brevibacterium, der hohe biosynthetische Aktivität für Valin, Leucin oder Isoleucin oder deren Vorläufer aufweist, erhältlich ist.

**24.** Verfahren zur Herstellung von L-Valin, L-Leucin oder L-Isoleucin, das darin besteht, daß ein in einem der Ansprüche 6 bis 22 definierter Mikroorganismus gezüchtet wird und die betreffende Aminosäure aus der Kulturflüssigkeit gewonnen wird.

**25.** Verfahren zur Herstellung von L-Valin oder L-Isoleucin nach Anspruch 24, wobei der Mikroorganismus Brevibacterium flavum FERM-P 10142 (FERM-BP 2516), Brevibacterium flavum FERM-P 10143 (FERM-BP 2509) oder Brevibacterium lactofermentum FERM P-10141 (FERM-BP 2508) ist.

**Revendications**

**1.** Plasmide ou phage capable de multiplication autonome dans lequel est incorporé le gène de l'acétohydroxyacide synthétase dérivé d'un microorganisme appartenant au genre Brevibacterium.

**2.** Plasmide ou phage selon la revendication 1 dans lequel ledit gène de l'acétohydroxyacide synthétase est un gène de type sauvage.

**3.** Plasmide ou phage selon la revendication 1 dans lequel ledit gène de l'acétohydroxyacide synthétase est un gène muté débarrassé d'inhibition en retour.

**4.** Plasmide ou phage selon la revendication 1 dans lequel ledit gène de l'acétohydroxyacide synthétase est sous le contrôle de son promoteur inhérent ou d'un promoteur pour un autre type de gène.

**5.** Plasmide ou phage selon la revendication 1 dans lequel ledit gène de l'acétohydroxyacide synthétase est sous le contrôle de son promoteur inhérent ou d'un promoteur pour un autre type de gène résistant au triméthoprime.

**6.** Microorganisme portant un plasmide ou phage selon l'une quelconque des revendications 1 à 5.

**7.** Microorganisme selon la revendication 6, ledit microorganisme étant une bactérie Coryneforme productrice de glutamate.

8. Microorganisme selon l'une quelconque des revendications 6 ou 7, lequel microorganisme appartient au genre Brevibacterium.

9. Microorganisme selon la revendication 8, lequel microorganisme appartient à une espèce choisie parmi Brevibacterium flavum et Brevibacterium lactofermentum.

10. Microorganisme selon l'une quelconque des revendications 6 à 9, lequel microorganisme est capable de produire la valine, la leucine ou l'isoleucine.

11. Microorganisme selon l'une quelconque des revendications 6 à 10, lequel microorganisme est exempt de répression de la biosynthèse de la valine, de la leucine ou de l'isoleucine.

12. Microorganisme selon l'une quelconque des revendications 6 à 11, ledit microorganisme étant Brevibacterium lactofermentum.

13. Microorganisme selon la revendication 12, ledit microorganisme étant Brevibacterium lactofermentum FERM-P 10141 (FERM-BP 2508).

14. Microorganisme selon l'une quelconque des revendications 6 à 11, ledit microorganisme étant Brevibacterium flavum.

15. Microorganisme selon la revendication 14, ledit microorganisme étant Brevibacterium flavum FERM-P 10142 (FERM-BP 2516) ou Brevibacterium flavum FERM-P 10143 (FERM-BP 2509).

16. Microorganisme selon l'une quelconque des revendications 7, 10 ou 11, ledit microorganisme étant Corynebacterium glutamicum.

17. Microorganisme contenant le gène de l'acétohydroxyacide synthétase, ledit gène ayant été transféré du plasmide selon la revendication 1 au chromosome.

18. Microorganisme selon la revendication 17, ledit microoganisme étant Brevibacterium lactofermentum.

19. Microorganisme selon la revendication 17, ledit microorganisme étant Brevibacterium flavum.

20. Microorganisme selon la revendication 17, ledit microorganisme étant Corynebacterium glutamicum.

21. Microorganisme selon l'une quelconque des revendications 6 à 20 dans lequel est contenu un plasmide ou phage supplémentaire qui a pour effet d'augmenter le rendement en valine, leucine ou isoleucine.

22. Microorganisme selon la revendication 21 dans lequel le plasmide supplémentaire peut coexister avec le plasmide selon la revendication 1.

23. ADN recombiné comprenant le gène codant l'acétohydroxyacide synthétase, ledit gène codant l'acétohydroxyacide synthétase pouvant être obtenu à partir d'un microorganisme appartenant au genre Brevibacterium ayant une activité biosynthétique élevée pour la valine, la leucine ou l'isoleucine ou leurs précurseurs.

24. Procédé de production de L-valine, L-leucine ou L-isoleucine qui comprend la culture d'un microorganisme selon l'une quelconque des revendications 6 à 22 et la récupération de l'acide aminé voulu à partir du liquide de culture.

25. Procédé de production de L-valine ou L-isoleucine selon la revendication 24 dans lequel ledit microorganisme est Brevibacterium flavum FERM P-10142 (FERM-BP 2516), Brevibacterium flavum FERM P-10143 (FERM-BP 2509) ou Brevibacterium lactofermentum FERM P-10141 (FERM-BP 2508).

Fig. 1

— pAJ220.  Inserted DNA  H:HindⅢ. X:XbaI. C:ClaI
S:SalI. P:PstI.  ERV:EcoRV. ERI:EcoRI. B:BclI.